Europäisches Patentamt

European Patent Office

Office européen des brevets

(19)

(11) Veröffentlichungsnummer: **0 171 443**

**A1**

(12) **EUROPÄISCHE PATENTANMELDUNG**

(21) Anmeldenummer: 84109588.8

(22) Anmeldetag: 11.08.84

(51) Int. Cl.⁴: **C 07 C 43/29**
C 07 C 41/22, C 08 K 5/06

(43) Veröffentlichungstag der Anmeldung:
19.02.86 Patentblatt 86/8

(84) Benannte Vertragsstaaten:
BE DE FR IT NL

(71) Anmelder: Chemische Fabrik Kalk GmbH
Kalker Hauptstrasse 22 Postfach 91 01 57
D-5000 Köln 91(DE)

(72) Erfinder: Königstein, Otto, Dr.
Alfred Nobel-Strasse 17
D-5000 Köln 90(DE)

(72) Erfinder: Kerscher, Utto, Dr.
Am Beissel 25
D-5042 Erftstadt(DE)

(72) Erfinder: Neukirchen, Ernst
Vorsterstrasse 75
D-5000 Köln 91(DE)

(72) Erfinder: Büttgens, Walter
Petersbergstrasse 2
D-5340 Bad Honnef 1(DE)

(54) Kernbromierte Gemische von Dimethyl-diphenyl-äther-Isomeren, deren Herstellung und deren Verwendung als Brandschutzmittel für Kunststoffe.

(57) Die Erfindung betrifft kernbromierte Gemische von Dimethyl-diphenyläther-Isomeren mit 4 bis 8 Bromatomen im Molekül. Hergestellt werden diese Verbindungen durch Umsetzung eines Dimethyl-diphenyläther-Isomerengemisches mit der für 4 bis 8 Bromatome im Molekül erforderlichen Menge rom in einem Lösemittel in Gegenwart eines Halogenierungskatalysators. Es werden dabei bromierte Gemische von Dimethyl-diphenyläther-Isomeren erhalten, die jeweils zur Hauptmenge aus Tetrabrom-, Pentabrom-, Hexabrom-, Heptabrom- und Octabromdimethyl-diphenyläther bestehen. Diese Produkte finden Verwendung als Brandschutzmittel für Styrol- oder Styrolcopolymerisate sowie für Phenolharzlaminate.

Gegenstand der Erfindung sind Gemische von kernbromierten Dimethyl-diphenyläther-Isomeren, ein Verfahren zu
deren Herstellung und deren Verwendung als Brandschutzmittel für Styrol- und Styrolcopolymerisate sowie für
Phenolharzlaminate.

Es ist bekannt, bromierte Diphenyle oder Diphenyläther
mit unterschiedlichem Bromierungsgrad herzustellen und
als Brandschutzkomponente für Kunststoffe zu verwenden.

In der CH-PS 569 042 werden bromierte Dialkyl-diphenyläther beschrieben, die an den beiden Benzolkernen mit
jeweils einer Alkylgruppe mit 1-4 C-Atomen und jeweils
4 Bromatomen am Kern substituiert sind, also Dialkyloctabromdiphenyläther darstellen. Am Beispiel des Dimethyl-
octabrom-diphenyläthers wird die Verwendbarkeit dieser
Verbindung als Brandschutzmittel für Polyester- oder Polyamidfasern beschrieben.

Dieser Octabromdimethyl-diphenyläther wird hergestellt
durch Eintropfen einer Lösung von 4,4'-Dimethyl-diphenyläther, der in 1,2-Dichloräthan gelöst ist, in das in 10-
%igem Überschuß vorgelegte Brom in Gegenwart von Eisenpulver als Katalysator. Das hochbromierte Produkt hat
einen Schmelzbereich von 258-262°C.

Bei der großtechnischen Herstellung von Kresolen fällt
als Nebenprodukt ein Isomerengemisch von dimethylierten
Diphenyläthern an, das für weitere chemische Umsetzungen
bereitsteht.

Aufgabe der vorliegenden Erfindung war es, aus einem Dimethyl-diphenyläther-Isomerengemisch durch Kernbromierung bromhaltige Dimethyl-diphenyläther mit unterschiedlichem Bromierungsgrad herzustellen und deren Verwendbarkeit als Brandschutzkomponente für Kunststoffe entsprechend den geforderten DIN-Normen nachzuweisen.

Gegenstand der Erfindung sind Gemische von kernbromierten Dimethyl-diphenyläther-Isomeren mit 4 bis 8 Bromatomen im Molekül und einem Bromgehalt von 60 bis 77 %.

Die Herstellung dieser Gemische von kernbromierten Dimethyl-diphenyläther-Isomeren erfolgt durch Umsetzung eines Isomerengemisches dimethylierter Diphenyläther in Gegenwart eines Halogenierungskatalysators in einem organischen Lösemittel mit Brom. Das Herstellungsverfahren ist dadurch gekennzeichnet. daß man ein Dimethyl-diphenyläther-Isomerengemisch mit der für 4 bis 8 Bromatome im Molekül erforderlichen äquimolaren Menge Brom in einem halogenierten Lösemittel und einem Halogenierungskatalysator bei Temperaturen zwischen 10 und 50° C umsetzt. Es werden Gemische von kernbromierten Dimethyl-diphenyläther-Isomeren erhalten, die jeweils zur Hauptmenge aus Tetrabrom-, Pentabrom-, Hexabrom-, Heptabrom- und Octabrom-dimethyl-diphenyläther bestehen.

Vorteilhaft wird bei der Herstellung dieser Produkte als Lösemittel ein halogenierter Kohlenwasserstoff, wie beispielsweise 1,1,2,2-Tetrachloräthan, verwendet und als Halogenierungskatalysator kann sowohl Al-chlorid als auch Eisenpulver verwendet werden.

Hergestellt werden die erfindungsgemäßen Produkte, indem das Gemisch der Dimethyl-diphenyläther-Isomeren in dem halogenierten Lösemittel gelöst und in Gegenwart des Halogenierungskatalysators durch Zugabe der dem gewünschten Bromierungsgrad entsprechenden äquimolaren Menge Brom
umgesetzt wird. Die Reaktionstemperaturen liegen bei der
Verwendung von Al-chlorid als Katalysator vorzugsweise
bei 10-20°C bzw. bei der Verwendung von Eisenpulver bei
40-50°C

Je nach dem Grad der Bromierung haben die Gemische von
kernbromierten Dimethyl-diphenyläther-Isomeren unterschiedliche Schmelzbereiche. So haben der Tetrabrom- und der
Pentabrom-dimethyl-diphenyläther bei Raumtemperatur eine
harzartige Konsistenz, während die höher bromierten Dimethyl-diphenyläther-Isomeren Schmelzbereiche von 160-250°C
haben.

Demzufolge muß auch die Aufarbeitung der harzartigen Produkte nach der beendeten Bromierungsreaktion anders vorgenommen werden, als die Aufarbeitung der höher bromierten Produkte, die als Feststoffe gewonnen werden.

Im Falle der harzartigen Produkte wird nach der Bromierungsreaktion das Reaktionsgemisch erst mit Wasser und anschließend mit verdünnter Sodalösung gewaschen und nach dem Trocknen der organischen Phase das Lösemittel unter vermindertem
Druck abdestilliert, wobei das jeweilige Reaktionsprodukt
als harziger Rückstand verbleibt.

Bei den höher bromierten Produkten scheiden sich diese während der Bromierungsreaktion als Feststoff ab, der aus dem
Reaktionsgemisch isoliert und nach anschließendem Waschen mit einem alkoholischen Lösemittel, wie beispielsweise
Methanol, in feinteiliger kristalliner Form erhalten wird.

Die auf diese Weise gewonnenen erfindungsgemäßen Produkte
können allein oder deren Gemische gegebenenfalls unter
Mitverwendung von anderen brom- oder phosphorhaltigen Verbindungen, gegebenenfalls unter Zusatz von Antimontrioxid,
als Brandschutzkomponenten für Kunststoffe eingesetzt werden.

So werden vorteilhaft die höher bromierten Produkte, allein
oder deren Gemische, deren Schmelzbereiche zwischen 160
und 260°C liegen, in Mengen von 10 bis 30 Gew%, vorzugsweise von 15 bis 25 Gew%, bezogen auf die Polymermasse,
zur Brandschutzausrüstung von Styrol- oder Styrolcopolymerisaten verwendet werden. Dazu werden diese erfindungsgemäßen Produkte im Temperaturbereich von 200-270°C unter
Mitverwendung von Antimontrioxid in die Polymermatrix eingebaut. Die mechanischen und physikalischen Eigenschaften
der mit diesen höher bromierten Produkten ausgerüsteten
Polymermassen werden dabei nur unwesentlich beeinflußt.

Überraschenderweise hat sich gezeigt, daß diese höher bromierten Produkte bei der thermischen Verarbeitung der damit ausgerüsteten Styrolpolymerisate ein wesentlich günstigeres Schmelzverhalten bewirken als andere vergleichbare
Brandschutzkomponenten. Die Viskosität der mit den erfindungsgemäßen Produkten ausgerüsteten Polymerisate ist bei
den erforderlichen Verarbeitungstemperaturen erheblich
niedriger, wie aus dem Vergleich der Schmelzindizes von
Styrolpolymerisaten, die die erfindungsgemäßen kernbromierten Gemische von Dimethyl-diphenyläther-Isomeren enthalten,
und Styrolpolymerisaten mit herkömmlichen Brandschutzmitteln hervorgeht. Eine Gegenüberstellung der Schmelzindizes ist der nachfolgenden Tabelle 2 zu entnehmen.

Für Hartpapiere auf Phenolharzbasis, sogenannte Phenolharzlaminate, wird bei Verwendungszwecken, die einer erhöhten Brandgefahr unterliegen, eine Brandbeständigkeit gefordert, die dem Brandtest nach NEMA LI-1965, Klasse FR 2 und dem Brandtest nach UL 94, Klasse V-O entspre-chen muß.

Zur Brandschutzausrüstung dieser Phenolharzlaminate werden vorteilhaft die harzartigen bromierten Produkte der Erfindung, insbesondere Gemische von kernbromierten Dimethyl-diphenyläther-Isomeren mit 67 bis 68 % Brom und einem Schmelzbereich von 50-65 °C, verwendet. Diese bei Raumtemperatur harzartigen Produkte haben besonders günstige Löse- und Verarbeitungseigenschaften für Phenol- oder Kresolharze.

Mit den nachfolgenden Beispielen soll die Herstellung der erfindungsgemäßen Produkte und deren Verwendbarkeit als Brandschutzmittel näher erläutert werden.

Beispiel 1  Tetrabrom-dimethyl-diphenyläther (Herstellung)
─────────────────

In einem Reaktionsgefäß werden 560 Gewichtsteile (GT) 1,1,2,2-Tetrachloräthan, 198 GT Dimethyl-diphenyläther und 10 GT Al-chlorid vorgelegt. Unter Rühren werden im Laufe von 3 Stunden 640 GT Brom zudosiert. Durch Außenkühlung mit Wasser wird dabei die Reaktionstemperatur auf 15-20 °C gehalten. Nach beendeter Bromierungsreaktion wird das Reaktionsgemisch zunächst mit 250 GT Wasser und anschließend mit 250 GT 3%iger Sodalösung gewaschen. Die organische
Phase wird mit 10 GT Bleicherde getrocknet, filtriert
und bei 12 Torr. abdestilliert. Letzte Reste von Tetrachloräthan werden bei einer Sumpftemperatur von 100°C
und 15 Torr. entfernt.

Es werden 486 GT Tetrabrom-dimethyl-diphenyläther als
braunes Harz mit einem Bromgehalt von 61,7 % erhalten.
Die Ausbeute beträgt 94,6 % der Theorie.
Nach GC-Analyse besteht das Produkt aus 77 % Tetrabrom-
und 11 % Pentabrom-dimethyl-diphenyläther (neben weiteren
nicht identifizierbaren Komponenten.)

Beispiel 2   Pentabrom-dimethyl-diphenyläther (Herstellung)

Analog dem Beispiel 1 werden 600 GT Tetrachloräthan, 198
GT Dimethyl-diphenyläther und 0,7 GT Fe-Pulver vorgelegt
und im Laufe von 6 Stunden mit 800 GT Brom bei einer Reaktionstemperatur von 40-50°C umgesetzt. Die Aufarbeitung
erfolgt analog dem Beispiel 1.

Es werden 490 GT Pentabrom-dimethyl-diphenyläther als festes Harz mit einem Bromgehalt von 66,6 % und einem Schmelzbereich von 50-65°C erhalten. Die Ausbeute beträgt 83 %
der Theorie.

Nach GC-Analyse besteht das Produkt aus 26,5 % Tetrabrom-
dimethyl-, 60,5 Pentabrom- und 13,0 % Hexabrom-dimethyl-
diphenyläther Beispiel 3   Hexabrom-dimethyl-diphenyläther (Herstellung)

Analog dem Beispiel 1 werden 480 GT Tetrachloräthan, 198
GT Dimethyl-diphenyläther und 8 GT Al-chlorid vorgelegt
und im Laufe von 4 Stunden mit 767 GT Brom bei einer Reaktionstemperatur von 10-15°C umgesetzt. Das während der
Reaktion ausgefallene Festprodukt wird abgetrennt, mit
100 GT Methanol gewaschen und bei einer Temperatur von
90°C getrocknet.

Es werden 441 GT Hexabrom-dimethyl-diphenyläther als weißliches kristallines Produkt mit einem Schmelzbereich von
162-167°C und einem Bromgehalt von 71,3 % erhalten. Die
Ausbeute beträgt 87 % der Theorie.
Nach GC-Analyse besteht das Produkt aus 5,1 % Pentabrom-,
75,5 % Hexabrom- und 19,4 % Heptabrom-dimethyl-diphenyläther.

Beispiel 4   Heptabrom-dimethyl-diphenyläther (Herstellung)

Analog dem Beispiel 1 werden 480 GT Tetrachloräthan, 120
GT Dimethyl-diphenyläther und 6 GT Al-chlorid vorgelegt
und im Laufe von 4 Stunden mit 678 GT Brom bei einer Reaktionstemperatur von 10-15°C umgesetzt. Das während der
Reaktion ausgefallene Festprodukt wird abgetrennt, mit
200 GT Methanol gewaschen und bei einer Temperatur von
90°C getrocknet.

Es werden 393 GT Heptabrom-dimethyl-diphenyläther als kristallines Produkt mit einem Schmelzbereich von 242-248°C
und einem Bromgehalt von 73,5 % erhalten. Die Ausbeute
beträgt 87 % der Theorie.

Nach GC-Analyse besteht das Produkt aus 2,0 Hexabrom-,
97,4 % Heptabrom- und 0,5 % Octabrom-dimethyldiphenyläther.


Beispiel 5   Octabrom-dimethyl-diphenyläther (Herstellung)

Analog dem Beispiel 1 werden 900 GT Tetrachloräthan, 198
GT Dimethyl-diphenyläther und 10 GT Al-chlorid vorgelegt
und im Laufe von 5 Stunden bei einer Reaktionstemperatur
von 10-15°C mit 1280 GT Brom umgesetzt. Das während der
Reaktion ausgefallene Festprodukt wird abgetrennt, mit
800 GT Methanol 3 Stunden unter Rühren am·Rückfluß erhitzt,
nach dem Abkühlen wieder abgetrennt und bei 90°C getrocknet.

Es werden 754 GT Ocatbom-dimethyl-diphenyläther als kristallines Produkt mit einem Schmelzbereich von 254-260°C
und einem Bromgehalt von 76,2 % erhalten. Die Ausbeute beträgt 91 % der Theorie.
Nach GC-Analyse besteht das Produkt aus 9,3 % Heptabrom-
und 90,5 % Octabrom-dimethyl-diphenyläther.


Die gemäß den Herstellungsbeispielen 3, 4 und 5 beschriebenen Produkte, Hexabrom-, Heptabrom- und Octabrom-dimethyl-diphenyläther, mit Schmelzbereichen von 162-167°C
bzw. 242-248°C und 254-260°C werden bei der Verarbeitung
von Styrol- bzw. Styrolcopolymerisaten im Temperaturbereich von 200-270°C in die Polymermatrix eingebaut.

Beispiel 6   (Verwendung)

In einem Pflugbettmischer wird das als Granulat vorliegende, mit Polybutadien modifizierte, schlagzähe Polystyrol (HIPS) bzw. Acrylnitril-Butadien-Styrol-Polymerisat
(ABS) eingefüllt. Unter Mischen wird ein aus 70 Gew% Pentabromdiphenyläther und 30 Gew% Diphenylkresylphosphat
bestehendes Gemisch als Haftvermittler, die kernbromierten
Dimethyl-diphenyläther-Isomeren und Antimontrioxid zugegeben. Nach 5 Minuten Mischzeit sind die pulverförmigen
Produkte fest auf den Oberflächen der Grananlien fixiert,
so daß die Mischung praktisch staubfrei ist.

Die Zusammensetzung dieser Polymermassen und Prüftests
sind in der nachfolgenden Tabelle 1 zusammengestellt. Wie
daraus zu ersehen ist, werden die mechanischen und physikalischen Eigenschaften der brandgeschützt ausgerüsteten
Polymermassen nur unwesentlich beeinflußt.

Bei den Versuchen A,B,C und E,F,G sind die erfindungsgemäßen Produkte eingebaut, während die Versuche D und H
nur Vergleichsversuche darstellen.

Tabelle 1

| Polymermischung | A | B | C | D | E | F | G | H |
|---|---|---|---|---|---|---|---|---|
| **1. <u>Zusammensetzung</u>** (Angaben in GT) | | | | | | | | |
| HIPS | 10 | 10 | 10 | 10 | - | - | - | - |
| ABS | - | - | - | - | 10 | 10 | 10 | 10 |
| Haftvermittler | 0,1 | 0,1 | 0,1 | - | 0,1 | 0,1 | 0,1 | - |
| Hexabromverb. (nach Beispiel 3) | 2,2 | - | - | - | 2,3 | - | - | - |
| Heptabromverb. (nach Beispiel 4) | - | 2,1 | - | - | - | 2,2 | - | - |
| Octabromverb. (nach Beispiel 5) | - | - | 1,7 | - | - | - | 1,9 | - |
| $Sb_2O_3$ | 0,65 | 0,63 | 0,6 | - | 0,7 | 0,65 | 0,63 | - |
| **2. <u>Prüftests</u>** | | | | | | | | |
| UL 94 | VO | VO | VO | HB | VO | VO | VO | HB |
| Zugfestigkeit, N/mm² DIN 53455 | 21 | 21 | 22 | 22 | 44 | 44 | 43,5 | 45 |
| Reißfestigkeit, N/mm² DIN 53455 | 19,5 | 19 | 19,5 | 20 | 33 | 33 | 32 | 34 |
| Reißdehnung, m/m DIN 53455 | 0,3 | 0,3 | 0,25 | 0,4 | 0,15 | 0,14 | 0,2 | 0,2 |
| Schlagzähigkeit, KJ/m² DIN 53453 | 65 | 63 | 40 | ohne Bruch | 92 | 97 | 58 | ohne Bruch |
| Wärmeformbeständigkeit, °C DIN 53461 | 84 | 85 | 88 | 89 | 92 | 93 | 97 | 98 |

Tabelle 2

| Zusammensetzung der Polymermasse | Schmelzindex MFI 200°C / 5 Kp |
|---|---|
| 1. erfindungsgemäß<br><br>10 GT HIPS<br>0,1 " Haftvermittler<br>1,7 " Octabrom-Verb. (nach Beispiel 5)<br>0,6 " Sb$_2$O$_3$ | 10,1 – 10,8 g/10 Min |
| 2. Vergleich<br><br>10 GT HIPS<br>0,1 " Haftvermittler<br>1,6 " Decabromdiphenyl-äther<br>0,6 " Sb$_2$O$_3$ | 7,4 – 7,7 g/ 10 Min |

Zur Brandschutzausrüstung von Phenolharzlaminaten wird
der entsprechend dem Beispiel 2 hergestellte bromierte
Dimethyl-diphenyläther eingesetzt, dessen Hauptbestandteil neben Tetrabrom- und Hexabrom-dimethyl-diphenyläther
Pentabrom-dimethyl-diphenyläther ist und dessen Bromgehalt etwa 66,6 % beträgt.

Die flammhemmenden Komponenten werden in der harzhaltigen Imprägnierlösung gelöst oder in einem geeigneten Lösemittel, wie Aceton oder Toluol, als 80-95 %ige Lösung
der Imprägnierlösung zugesetzt. Diese Imprägnierharzlösungen können auf Laminierungsanlagen konventioneller
Bauart ohne Anlagenveränderung verarbeitet werden. Als
Trägermaterial kommen sowohl organische Stoffe, wie Baumwollgewebe, Papiere und Holzfurniere, als auch anorganische Stoffe, wie Glasseide, zur Anwendung. Die so hergestellten Schichtpreßplatten finden vornehmlich Verwendung
auf dem Elektrosektor oder als schwerbrennbar eingestellte Dekor- oder Verbundplatten in verschiedenen Anwendungsbereichen.

Beispiel 7   (Verwendung)

In der nachfolgenden Tabelle 3 sind die erfindungsgemäßen
Tränkharzmischungen zusammengestellt und die jeweiligen
Brandtests der damit hergestellten Laminatplatten aufgeführt, wobei der Versuch A einen Vergleichsversuch ohne
Brandschutzausrüstung darstellt.

Tabelle 3

| Laminatplatte | Brandtests | | |
|---|---|---|---|
| | ASTM-D 635 | UL 94 | NEMA-Norm |
| **Zusammensetzung der Tränkharzmischung (Angaben in Gew%)** | | | |
| A  (Vergleich)<br><br>83  Kresolharz<br>17  Phenolharz | 150 sec/<br>127 mm | HB | wird nicht<br>erfüllt |
| B  (erfind.gem.)<br><br>55  Kresolharz<br>11  Phenolharz<br>20  Brom-Produkt<br>14  Diphenyl-<br>    kresylphosphat | 2 sec/<br>15 mm | VO | FR 2 |
| C  (erfind.gem)<br><br>55  Phenolharz<br>11  Kresolharz<br>20  Brom-Produkt<br>22  Diphenyl-<br>    kresylphosphat | 3 sec/<br>18 mm | VO | FR 2 |
| D  (erfind.gem)<br><br>50  Kresolharz<br>10  Phenolahrz<br>18  Brom-Produkt<br>22  Diphenyl-<br>    octylphosphat | 4 sec/<br>16 mm | VO | FR 2 |

- 1 -

0171443

Patentansprüche

1. Kernbromierte Gemische von Dimethyl-diphenyläther-
   Isomeren mit 4 bis 8 Bromatomen im Molekül und einem
   Bromgehalt von 60 bis 77 %.

2. Verfahren zur Herstellung der kernbromierten Gemische
   von Dimethyl-diphenyläther-Isomeren nach Anspruch 1
   durch Umsetzung eines Isomerengemisches dimethylierter Diphenyläther in Gegenwart eines Halogenierungskatalysators in einem organischen Lösemittel mit Brom,
   dadurch gekennzeichnet, daß man ein Dimethyl-diphenyläther-Isomerengemisch mit der für 4 bis 8 Bromatome
   im Molekül erforderlichen äquimolaren Menge Brom in
   einem halogenierten Lösemittel und einem Halogenierungskatalysator bei Temperaturen zwischen 10 und 50°C
   umsetzt, und bromierte Dimethyl-diphenyläther-Isomere
   erhalten werden, die jeweils zur Hauptmenge aus Tetrabrom-, Pentabrom-, Hexabrom-, Heptabrom- und Octabrom-
   dimethyl-diphenyläther bestehen.

3. Verfahren nach Anspruch 2, dadurch gekennzeichnet,
   daß man als halogeniertes Lösemittel 1,1,2,2-Tetrachloräthan verwendet.

4. Verfahren nach Ansprüchen 2 und 3, dadurch gekennzeichnet, daß man als Halogenierungskatalysator Al-
   chlorid oder Eisenpulver verwendet.

5. Verfahren nach Ansprüchen 2 bis 4, dadurch gekennzeichnet, daß die erhaltenen Gemische von kernbromierten Dimethyl-diphenyläther-Isomeren einen Bromgehalt von 62,0 bis 63,0 % haben.

6. Verfahren nach Ansprüchen 2 bis 4, dadurch gekennzeichnet, daß die erhaltenen Gemische von kernbromierten Dimethyl-diphenyläther-Isomeren bei einem
Bromgehalt von 67,0 bis 68,0 % einen Schmelzbereich
von 50-65°C haben.

7. Verfahren nach Ansprüchen 2 bis 4, dadurch gekennzeichnet, daß die erhaltenen Gemische von kernbromierten Dimethyl-diphenyläther-Isomeren bei einem
Bromgehalt von 71,0 bis 72,0 % einen Schmelzbereich
von 160-190°C haben.

8. Verfahren nach Ansprüchen 2 bis 4, dadurch gekennzeichnet, daß die erhaltenen Gemische von kernbromierten Dimethyl-diphenyläther-Isomeren bei einem
Bromgehalt von 74,0 bis 75,0 % einen Schmelzbereich
von 215-250°C haben.

9. Verfahren nach Ansprüchen 2 bis 4, dadurch gekennzeichnet, daß die erhaltenen Gemische von kernbromierten Dimethyl-diphenyläther-Isomeren bei einem
Bromgehalt von 76,0 bis 77,1 % einen Schmelzbereich
von 250-265°C haben.

10. Verwendung der kernbromierten Gemische von Dimethyl-
diphenyläther-Isomeren nach Anspruch 1 als Brandschutzkomponente für Kunststoffe.

11. Verwendung der kernbromierten Gemische von Dimethyl-
diphenyläther-Isomeren nach Anspruch 1 mit einem Bromgehalt von 71,0 bis 77,1 % als Brandschutzmittel
für Styrol- und Styrolcopolymerisate.

12. Verwendung der kernbromierten Gemische von Dimethyl-
diphenyläther-Isomeren nach Anspruch 11, dadurch gekennzeichnet, daß man diese in Mengen von 10 bis 30
Gew%, vorzugsweise von 15 bis 25 Gew%, bezogen auf
das Styrol- oder Styrolcopolymerisat, unter Mitverwendung von vorzugsweise 5 bis 10 Gew% Antimontrioxid einsetzt.

13. Verwendung der kernbromierten Gemische von Dimethyl-
diphenyläther-Isomeren nach Anspruch 1 mit einem Bromgehalt von 62 bis 72 % als Brandschutzmittel für Phenolharzlaminate.

14. Verwendung der kernbromierten Gemische von Dimethyl-
diphenyläther-Isomeren nach Anspruch 13, dadurch gekennzeichnet, daß man sie in Mengen von 3 bis 25
Gew%, vorzugsweise von 10 bis 20 Gew%, bezogen auf
die Tränkharzmischung, einsetzt.

0171443
Nummer der Anmeldung

EP 84 10 9588

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | GB-A-1 364 397 (INVENTA)<br><br>* Ansprüche; Beispiele; Seite 2, Zeilen 23-68 * & CH - A - 569 042 (Kat. D,X) | 1-4,9-12 | C 07 C 43/29<br>C 07 C 41/22<br>C 08 K 5/06 |

-----

RECHERCHIERTE
SACHGEBIETE (Int Cl 4)

C 07 C 41/00
C 07 C 43/00

Der vorliegende Recherchenbericht wurde für alle Patentansprüche erstellt

| Recherchenort<br>DEN HAAG | Abschlußdatum der Recherche<br>02-04-1985 | Prüfer<br>WRIGHT M.W. |
|---|---|---|